**Europäisches Patentamt**

**European Patent Office**

**Office européen des brevets**

(19)

(11) Veröffentlichungsnummer : **0 326 826 B1**

(12) # EUROPÄISCHE PATENTSCHRIFT

(45) Veröffentlichungstag der Patentschrift :
**25.03.92 Patentblatt 92/13**

(21) Anmeldenummer : **89100493.9**

(22) Anmeldetag : **12.01.89**

(51) Int. Cl.[5] : **A61K 33/42,** A61K 33/30, A61K 33/00, A61K 31/34, A61K 31/19, // (A61K33/42, 33:30, 33:00, 31:70, 31:34, 31:19, 31:11)

(54) **Mittel mit zerstörender Wirkung auf maligne Tumore, Verfahren zu dessen Herstellung und Präparation zur Anwendung in der Therapie von Krebskranken.**

(30) Priorität : **03.02.88 AT 218/88**

(43) Veröffentlichungstag der Anmeldung :
**09.08.89 Patentblatt 89/32**

(45) Bekanntmachung des Hinweises auf die Patenterteilung :
**25.03.92 Patentblatt 92/13**

(84) Benannte Vertragsstaaten :
**AT BE CH DE ES FR GB GR IT LI LU NL SE**

(56) Entgegenhaltungen :
**CHEMICAL ABSTRACTS, Band 93, Nr. 17, 27. Oktober 1980, Seiten 466-467, Zusammenfassung Nr. 165706t, Columbus, Ohio, US; L. KRONBERGER et al.: "Tumor host relations. VI. Is alpha-ketoglutarate a tumor marker? Association with tumor extent in humans-correlation with tumor size in rats", & J. CANCER RES. CLIN. ONCOL. 1980, 97(3), 295-9**
**CHEMICAL ABSTRACTS, Band 93, Nr. 7, 18. August 1980, Seite 688, Zusammenfassung Nr. 68178q, Columbus, Ohio, US; W. SCHREIB-MAYER et al.: "Tumor host relations. IV. Tissue distribution of citric acid cycle intermediates and of glutamated in tumor-bearing rats high level of alpha-ketoglutarate in solid Yoshida sarcomas", & J. CANCER RES. CLIN. ONCOL. 1980, 97(2), 137-44**
**CHEMICAL ABSTRACTS, Band 90, Nr. 25, 18. Juni 1979, Seite 449, Zusammenfassung Nr. 201963y, Columbus, Ohio, US; R.J. SCHAUR et al.: "Tumor host relations. III. Increase of alpha-ketoglutarate in whole blood and urine and hypoalbuminemia of rats bearing the solid rhabdomyosarcoma BA 1112 and the ascitic or solid forms of Walker-carcinoma 256 and Yoshida sarcoma", & J. CANCER RES. CLIN. ONCOL. 1979, 93(3), 293-300**

(56) Entgegenhaltungen :
**CHEMICAL ABSTRACTS, Band 80, Nr. 21, 27. Mai 1974, Seite 286, Zusammenfassung Nr. 118980z, Columbus, Ohio, US; M. BLINDER et al.: "Concentration of alpha-oxoglutaric acid at the various stages of the carcinoma of the cervix (of the uterus)", & ARCH. GYNAEKOL. 1973, 215(3), 315-24**

(73) Patentinhaber : **LEOPOLD PHARMA GESELLSCHAFT m.b.H.**
**Hafnerstrasse 36**
**A-8055 Graz (AT)**

(72) Erfinder : **Groke, Karl, Dr.**
**A-8063 Eggersdorf Nr.327 (AT)**
Erfinder : **Miggitsch, Hans, Dr.**
**Waltendorfer Hauptstrasse 181**
**A-8042 Graz (AT)**
Erfinder : **Musil, Horst, Dr.**
**Hans-Riehl-Gasse 8**
**A-8043 Graz (AT)**
Erfinder : **Polzer, Josef**
**Grottenhofstrasse 38**
**A-8053 Graz (AT)**

(74) Vertreter : **Karau, Wolfgang, Dr. et al**
**BASF Aktiengesellschaft Carl-Bosch-Strasse 38**
**W-6700 Ludwigshafen (DE)**

## Beschreibung

Die Erfindung betrifft ein Mittel mit zerstörender Wirkung auf maligne Tumore, ein Verfahren zu dessen Herstellung und eine Präparation zur Anwendung in der Therapie von Krebserkrankungen.

Es ist bekannt, daß bei Patienten mit malignen Tumoren eine hochsignifikante Vermehrung von alpha-Ketoglutarsäure im Blut feststellbar ist. Hierbei werden Blutspiegel von 0,30 mg % genannt, während der normale Blutspiegel mit 0,18 mg % angegeben wird. Dieser erhöhte Blutspiegel konnte durch Verabreichung von Kalium-Magnesium-Aspartat, allerdings nur für die Dauer der Verabreichung, normalisiert werden, wobei auch eine Besserung des Allgemeinzustandes des Patienten erzielt werden konnte (L. Kronberger und E. Fink, "Nachbehandlung operierter Karzinompatienten mit Aspartat", Wissenschaftliche Gesellschaft der Ärzte in der Steiermark, Vorträge vom 11.12.1970, Auszug aus den Sitzungsberichten).

Andererseits wurde von K. Schelstraete et al, The British J. of Radiology, Vol 55, Nr. 659 (1982), Seiten 797 bis 804 festgestellt, daß maligne Tumore eine durch Positron Emission Computer Tomographie bestimmbare vermehrte Aufnahme von $^{13}NH_3$ zeigen, die nach erfolgreicher Behandlung solcher Tumoren zurückgeht. Diese erhöhte $^{13}NH_3$-Aufnahme ist beträchtlich und beträgt bei manchen malignen Tumoren vier bis fünfmal so viel wie bei normalen Geweben.

Es ist auch bekannt, daß in einer Reihe von malignen Tumoren ein Gehalt an biogenen Aminen, wie z.B. Dopamin, 5-Hydroxytryptamin, Epinephrin und Norepinephrin nachweisbar sind, die aus Precursor-Substanzen durch Decarboxylierung entstehen. (R. Narotzky und W. Bondareff, The J. of Cell biology, 63, 1974, 64-70; L.V. Bader, A.W.J. Lykke und H. Hinterberger, Pathology 9, 1977, 353-358).

Der vorliegenden Erfindung liegt die Erkenntnis zugrunde, daß der erhöhte Blutspiegel an der alpha-Ketoglutarsäure auf eine Störung des Zitronensäurecyclus in den Zellen des Tumors zurückzuführen sein könnte und diese wegen ihres stark sauren pH-Wertes für den Tumor schädliche Substanz aus dem Tumorgewebe ausgeschieden wird, wozu der in solchen Tumoren in erhöhtem Ausmaß vorhandene Ammoniak bzw. die vorhandenen Amine dienen.

Überraschenderweise konnte darauf aufbauend gefunden werden, daß bei Verabreichung von größeren Mengen an alpha-Ketoglutarsäure an Patienten mit malignen Tumoren zwecks Erzielung eines Serumspiegels an dieser Substanz, der dann über jenem liegt, der bei solchen Patienten gefunden wird, bei gleichzeitiger Verabreichung einer wasserlöslichen, physiologisch verträglichen Substanz, die in enzymunabhängiger Reaktion Ammoniak und Amine unter Azomethinbildung zu binden vermag, diese offenbar dem Selbstschutz des Tumors dienende Ausscheidungsreaktion zum Erliegen kommt und eine Anreicherung an alpha-Ketoglutarsäure im Tumor die Folge ist, was zu einer bis zur völligen Eliminierung des Tumors führenden Schädigung desselben führt. Als Substanzen, die die Forderung nach einer Bindung von Ammoniak und Aminen in idealer Weise erfüllen, haben sich 5-Hydroxymethylfurfural, Dehydroascorbinsäure, Maltol und Vanillin erwiesen.

Gegenstand der vorliegenden Erfindung ist demnach ein Mittel mit zerstörender Wirkung auf maligne Tumore, das dadurch gekennzeichnet ist, daß es als Wirkstoffe alpha-Ketoglutarsäure und eine oder mehrere zur Azomethinbildung befähigte Verbindungen aus der Gruppe 5-Hydroxymethylfurfural, Dehydroascorbinsäure, Maltol und Vanillin enthält.

Das Mittel gemäß vorliegender Erfindung kann sowohl ein intravenös zu verabreichendes Mittel, vorzugsweise eine Infusion, als auch ein auf anderem Wege, wie z.B. ein oral oder rectal zu verabreichendes Mittel, oder in Fällen von Erkrankungen von nach außen geöffneten Körperhöhlen auch eine Spülung sein. Die Anwendung als Infusion ist deshalb sehr günstig, weil hiermit am verläßlichsten ein gleichmäßiger und hoher Blutspiegel an beiden Wirkstoffen erzielt wird, der gewährleistet, daß die erfindungsgemäße zerstörende Wirkung auf den Tumor voll entfaltet wird.

Oral zu verabreichende Formen wie Kapseln, Tabletten, aber auch Suspensionen oder Lösungen, sowie rectal zu verabreichende Formen bieten sich vor allem für die Nachbehandlung von bereits erfolgreich mit dem erfindungsgemäßen Mittel behandelten Patienten an, die bereits in häusliche Pflege entlassen wurden. Damit ist aber der Anwendungsbereich dieser Darreichungsformen keinesfalls erschöpft. Sie sind vielmehr auch dort zu empfehlen, wo sie einen direkten Zugang zum Krankheitsherd ermöglichen, wie dies z.B. bei malignen Erkrankungen des Verdauungstraktes, einschließlich des Colons, der Fall ist. Das gleiche gilt für Spülungen, die beispielsweise für die Mundhöhle Vorteile bieten können oder für salbenartige Zubereitungen, pastöse Auftragungen oder Tinkturen zur Behandlung von malignen Erkrankungen der Haut.

Da alpha-Ketoglutarsäure eine starke Säure ist, ist es erforderlich, diese Säure zur Vermeidung von Unverträglichkeitserscheinungen durch Zugabe von Alkalien so weit abzustumpfen, daß der pH-Wert des Mittels in einen physiologisch verträglichen Bereich zu liegen kommt. Die Anforderungen richten sich dabei nach der Darreichungsform, für die das Mittel bestimmt ist. In der Regel ist es zweckmäßig, einen pH-Wert von 4-6 einzustellen.

Eine Ausnahme bildet die orale Darreichungsform, bei der der pH-Wert deutlich niedriger liegen kann.

Für diese Einstellung des pH-Wertes sind jedoch alle jene Substanzen, nämlich Ammoniak und Amine, auszuschließen, die mit der Oxogruppe der zur Azomethinbildung befähigten Verbindungen reagieren würden, da damit die erfindungsgemäß zu entfaltende Wirkung dieser Verbindungen verloren gehen würde.

Im Falle einer festen Darreichungsform hat es sich als zweckmäßig erwiesen, die Abstumpfung der Ketoglutarsäure dadurch zu erreichen, daß man sie gleich ganz oder teilweise in Form eines ihrer Monosalze mit physiologisch verträglichen Kationen einsetzt.

Es hat sich als vorteilhaft erwiesen, im erfindungsgemäßen Mittel die alpha-Ketoglutarsäure gegenüber der zur Azomethinbildung befähigten Verbindung in einem gewichtsmäßigen Überschuß einzusetzen.

Zweckmäßig soll das Gewichtsverhältnis zwischen alpha-Ketoglutarsäure und der zur Azomethinbildung befähigten Verbindung im Bereich von 2 : 1 bis 12 : 1 liegen, wobei ein Gewichtsverhältnis von 3 : 1 bis 8 : 1 bevorzugt und ein solches von 6 : 1 besonders bevorzugt ist.

Es hat sich ferner als günstig erwiesen, dem erfindungsgemäßen Mittel ein Monosaccharid, insbesondere Glucose oder Fructose, zuzusetzen, da die sauren Metabolite desselben die Wirkung der alpha-Ketoglutarsäure auf den malignen Tumor unterstützen und überdies auch eine stabilisierende Wirkung auf das Mittel ausgeübt wird. Mittel, die nicht der intravenösen Verabreichung dienen, können anstelle von Monosacchariden auch Disaccharide enthalten, die ebenfalls stablisierend wirken, bei oral zu verabreichenden Mitteln darüberhinaus auch geschmacksverbessernd sind. Schließlich ist die Einverleibung der üblichen, für den Organismus nötigen Elektrolyte zweckmäßig, wobei deren Art und Zusammensetzung vorteilhafterweise so gewählt wird, daß gleichzeitig die erwünschte Einstellung des pH-Wertes erzielt wird. Als Ionen, die dem erfindungsgemäßen Mittel zweckmäßig zugeführt werden, sind als Kationen das Natrium-, Kalium-, Calcium-, Magnesium- und Zinkion, als Anionen das Chloridion und das Phosphation zu nennen, wobei letzteres durch die Wahl von primärem oder sekundärem Phosphat eine günstige Möglichkeit der pH-Regulierung bietet. Natürlich kann Phosphat auch in Form von Glycerophosphat in das Mittel eingebracht werden.

Als zur Azomethinbildung befähigte Verbindung ist 5-Hydroxymethylfurfural besonders bevorzugt.

Für die Formulierung des erfindungsgemäßen Mittels als Infusions- lösung hat sich beispielsweise folgende Zusammensetzung sehr bewährt:

| | | | |
|---|---|---|---|
| alpha-Ketoglutarsäure | 5 | – 20 | g/l |
| 5-Hydroxymethylfurfural | 1 | – 3 | g/l |
| Glucose | 20 | –100 | g/l |
| Natrium | 60 | –160 | mmol/l |
| Kalium | 15 | – 40 | mmol/l |
| Calcium | 3 | – 6 | mmol/l |
| Magnesium | 3 | – 6 | mmol/l |
| Zink | 0,03– | 0,1 | mmol/l |
| Chloridion | 10 | – 25 | mmol/l |
| Phosphation | 15 | – 30 | mmol/l |

wobei mit einer Zusammensetzung

| | |
|---|---|
| alpha-Ketoglutarsäure | 6,0 – 16,0 g/l |
| 5-Hydroxymethylfurfural | 1,0 – 2,5 g/l |
| Glucose | 20 – 50 g/l |
| Natrium | 70 –160 mmol/l |
| Kalium | 20 – 40 mmol/l |
| Calcium | 4 – 6 mmol/l |
| Magnesium | 4 – 6 mmol/l |
| Zink | 0,07– 0,1 mmol/l |
| Chloridion | 16 – 25 mmol/l |
| Phosphation | 20 – 30 mmol/l |

besonders gute Ergebnisse erzielbar waren.

Für die orale Verabreichung können sowohl feste Formen wie Kapseln oder Tabletten als auch Suspensionen, die beispielsweise durch Aufschlämmung von Granulaten entstehen, und schließlich auch Lösungen eingesetzt werden, wobei in den beiden letztgenannten Fällen die zur Azomethinbildung befähigte Verbindung keinen intensiven oder gar unangenehmen Geschmack besitzen sollte. Auch hier hat sich 5-Hydroxymethylfurfural besonders bewährt. Wie schon erwähnt, bietet die orale Form die Möglichkeit, direkt an Krankheitsherde im Verdauungstrakt heranzukommen, wobei es vorteilhaft ist, die Formulierung des Mittels je nach Einwirkungsort zu modifizieren. Während bei einem Karzinom im Magen Suspensionen oder Lösungen zur Behandlung sehr geeignet sind, hat sich für die Behandlung von Dünndarmcarcinom die Anwendung des erfindungsgemäßen Mittels in Form von Tabletten, die mit einem magensaftresistenten Überzug versehen sind, besonders bewährt. Karzinome des Colons können außer mit Infusionen auch mit einer Form des erfindungsgemäßen Mittels behandelt werden, das in Form eines Klistiers verabreicht wird. In diesem Fall ist es zweckmäßig, dem erfindungsgemäßen Mittel, das die Wirkstoffe gelöst enthält, ein Verdickungsmittel, wie z.B. Methylcellulose, Acetylcellulose oder Polyethylenglycol zuzusetzen, um Reizungen im Darm, die zum Ausstoß des Mittels führen könnten, möglichst zu vermeiden. Schließlich können von außen zugängliche Karzinome oder Metastasen lokal, etwa durch salbenartige Zubereitungen oder pastöse Auftragungen behandelt werden, wobei zweckmäßig Zusätze wie z. B. Dimethylsulfoxyd einverleibt werden, die den Durchgang der Wirkstoffe durch die Haut begünstigen.

Die Herstellung des erfindungsgemäßen Mittels gelingt, indem man die beiden Wirkstoffe auf in der Galenik übliche Weise in eine homogene Mischung überführt, die im Falle der Herstellung einer Lösung gleichzeitig mit einer Aufnahme in einem Verdünnungsmittel, vorzugsweise Wasser, hergestellt werden kann. Vor, während oder nach erfolgter Mischung und Aufnahme in einem Verdünnungsmittel oder Mischung mit einem Streckmittel, wird der pH-Wert durch Zuführung von Alkalien, nicht aber von Ammoniak oder Aminen, auf den gewünschten Wert eingestellt. Wird das erfindungsgemäße Mittel in Form einer wäßrigen Lösung, etwa als Infusionslösung, zubereitet, geht man vorzugsweise so vor, daß zuerst die alpha-Ketocarbonsäure und die anderen Bestandteile in Wasser gelöst werden und der pH-Wert bevorzugt auf den Bereich von 4-6 eingestellt wird, bevor jene Verbindung zugefügt wird, die zur Azomethinbildung befähigt ist. Im Falle der Herstellung einer festen Darreichungsform werden die Wirkstoffe und die übrigen Zusätze in üblicher Weise gemischt, gegebenenfalls auch gemeinsam granuliert. Die Einstellung des gewünschten pH-Bereiches kann in diesem Fall zweckmäßigerweise durch Einsatz eines entsprechenden Teiles oder der gesamten Menge der alpha-Ketoglutarsäure in Form eines ihrer Monosalze vorgenommen werden. Dazu bietet sich das Mono-Na-Salz besonders an, sehr zweckmäßig ist auch der Einsatz von Monosalzen des Kaliums, Magnesiums oder des Zinks, wobei auch Mischungen von verschiedenen Monosalzen verwendet werden können, um die Menge an den einzelnen Kationen den physiologischen Bedingungen anpassen zu können. Auch im Falle der festen Zubereitungen ist es bevorzugt, zunächst das Monosalz der alpha-Ketoglutarsäure oder dessen Mischung mit der freien Säure mit den übrigen Bestandteilen wie Elektrolyte, Streckmittel und gegebenenfalls auch Zucker zu mischen, bevor jene Verbindung zugesetzt wird, die zur Azomethinbildung befähigt ist. Es hat sich als günstig erwiesen, die Herstellung des erfindungsgemäßen Mittels, besonders dann, wenn es sich um eine Lösung handelt, unter Inertgasatmosphäre vorzunehmen, was helfen soll, oxydationsempfindliche Stoffe, wie z.B. 5-Hydroxymethylfurfural vor einer Aktivitätsverminderung zu schützen.

Bei der Anwendung des erfindungsgemäßen Mittels in der Therapie von Patienten mit malignen Tumoren an verschiedenen Organen wie Lunge, Bronchien, Brust, Blase, Magen, Haut und dergleichen konnte bei einer täglichen Verabreichung des erfindungsgemäßen Mittels über einen Zeitraum von einigen Wochen, beispielsweise etwa 1-2 Monate, bereits ein Stadium erzielt werden, in dem der zuvor deutlich sichtbare Tumor im Röntgenbild nicht mehr nachweisbar war und auch eventuell vorhandene Metastasen erfolgreich bekämpft waren. Hand in Hand mit diesem manifesten Behandlungserfolg ging auch eine entscheidende Besserung des Allgemeinzustandes der Patienten. Auffallend rasche Behandlungserfolge wurden dort registriert, wo sich das Krebsgeschehen noch in einem frühen Stadium befand.

Ein besonderer Vorzug des erfindungsgemäßen Mittels liegt außerdem darin, daß schädliche oder auch nur unangenehme Nebenwirkungen nicht beobachtet werden konnten. Die Tagesdosis in der eigentlichen Behandlungsphase beträgt in der Regel etwa 3-30 g alpha-Ketoglutarsäure und 1-5 g Verbindung, die zur Azomethinbildung befähigt ist, vorzugsweise 5-Hydroxymethylfurfural, wobei sich die Höhe der Taggesdosis innerhalb dieses Bereiches nach Schwere des Falles, jedoch auch nach dem Allgemeinzustand des Patienten richtet. In Fällen, in denen der Krankheitsherd nicht durch Erzeugung eines Blutspiegels an den erfindungsgemäß gewählten Wirkstoffen sondern durch direkte Einwirkung der Wirkstoffe auf den Krankheitsherd bekämpft werden soll, wie dies z.B. bei Erkrankungen im Gastrointestinaltrakt durch orale oder rectale Gabe möglich ist, kann die Tagesdosis in der Regel etwas niedriger gehalten werden. Sie beträgt dann meistens etwa 3-9 g alpha-Ketoglutarsäure und 0,5-1,5 g Verbindung, die zur Azomethinbildung befähigt ist.

Etwa die gleiche Dosis ist auch angezeigt, wenn im Zuge einer nicht stationär durchgeführten Infusionsbehandlung einzelne Tage, an denen eine Infusion nicht möglich ist, durch orale Gaben überbrückt werden sollen. Es ist günstig, nach Erreichen eines Behandlungserfolges eine Nachbehandlung des Patienten unter Verabreichung des erfindungsgemäßen Mittels in geringerer Tagesdosis, vorzugsweise in für die orale oder rectale Verabreichung geeigneter Form, anzuschließen, um den erreichten Erfolg zu stabilisieren. In diesem Fall ist es empfehlenswert, mit der oben genannten Dosierung für die lokale Anwendung zu beginnen. In den meisten Fällen kann dann die Dosis weiter reduziert werden, beispielsweise sogar bis zu einer Tagesdosis von 2,25 g alpha-Ketoglutarsäure und 0,375 g der Verbindung, die zur Azomethinbildung befähigt ist.

In den nachfolgenden Beispielen werden detaillierte Beispiele für behandlungsgerechte Formen des erfindungsgemäßen Mittels und deren Anwendung in der Krebstherapie gegeben, ohne die vorliegende Erfindung darauf beschränken zu wollen.

Beispiel 1:

6 g alpha-Ketoglutarsäure werden gemeinsam mit 50 g Glucose bei Raumtemperatur in 900 ml Wasser, das durch Begasen sauerstoffarm gemacht wurde, gelöst, worauf durch Zugabe von 2 g festem Natriumhydroxyd der pH-Wert der Lösung auf einen Wert über 4 gebracht wird. Nach Eintragen von 2 g 5-Hydroxymethylfurfural wird die resultierende Lösung auf 1 l aufgefüllt. Sie ist zum Einsatz als Infusionslösung zur Behandlung von Patienten mit malignen Tumoren geeignet.

Beispiel 2:

Aus folgenden Substanzen

| | |
|---|---|
| alpha-Ketoglutarsäure | 6,000 g/l |
| 5-Hydroxymethylfurfural | 2,000 g/l |
| Calciumchlorid.$2H_2O$ | 0,588 g/l |
| KOH 85 %ig | 1,320 g/l |
| $MgCl_2$.$6H_2O$ | 0,813 g/l |
| NaOH | 1,200 g/l |
| Natrium-Glycerophosphat.$5H_2O$ | 6,122 g/l |
| $ZnCl_2$ | 0,010 g/l |
| Glucose | 50,000 g/l |

wird ein Liter einer Lösung bereitet, indem man zunächst die alpha-Ketoglutarsäure in destilliertem Wasser bei einer Temperatur von etwa 50° C löst, das vorher durch Begasen sauerstoffarm gemacht wurde. In die resultierende Lösung werden dann nacheinander NaOH und KOH, die Elektrolyte sowie die Glucose eingetragen, wobei gleichzeitig ein pH-Wert von über 4 eingestellt wird. Der so erhaltenen klaren Lösung wird schließlich unter Rühren das 5-Hydroxymethylfurfural hinzugefügt. Man erhält so eine klare, leicht gelbliche Lösung, die pro Liter 6 g alpha-Ketoglutarsäure, 2 g 5-Hydroxymethylfurfural, 50 g Glucose sowie Elektrolyte in folgenden molaren Konzentrationen enthält:

| | |
|---|---|
| Na | 70,00 mmol/l |
| K | 20,00 mmol/l |
| Ca | 4,00 mmol/l |
| Mg | 4,00 mmol/l |
| Zn | 0,07 mmol/l |
| Chlorid | 16,00 mmol/l |
| Phosphat | 20,00 mmol/l |

Der pH-Wert der Lösung beträgt 4,90, die Lösung hat eine berechnete Osmolalität von 490 mosmol/kg $H_2O$. Sie wird zur Anwendung als Infusionslösung in Flaschen von 1/2 Liter Inhalt abgefüllt.

Beispiel 3:

wie in Beispiel 2 beschrieben wird aus

| | |
|---|---|
| alpha-Ketoglutarsäure | 16,000 g/l |
| 5-Hydroxymethylfurfural | 2,000 g/l |
| NaOH | 4,000 g/l |
| KOH 85 %ig | 2,640 g/l |
| Natrium-Glycerophosphat.$5H_2O$ | 9,184 g/l |
| $MgCl_2$.$6H_2O$ | 1,220 g/l |
| $CaCl_2$.$2H_2O$ | 0,883 g/l |
| $ZnCl_2$ | 0,014 g/l |
| Glucose | 20,000 g/l |

1 Liter einer Lösung bereitet, die neben 16 g alpha-Ketoglutarsäure, 2 g 5-Hydroxymethylfurfural und 20 g Glucose Elektrolyte in folgender molarer Konzentration enthält:

| | | |
|---|---|---|
| Natrium | 160 | mmol/l |
| Kalium | 40 | mmol/l |
| Calcium | 6 | mmol/l |
| Magnesium | 6 | mmol/l |
| Zink | 0,1 | mmol/l |
| Chlorid | 24,2 | mmol/l |
| Phosphat | 30 | mmol/l |

Der pH-Wert der Lösung beträgt 4,1, die berechnete Osmolalität etwa 530 mosmol/kg $H_2O$. Die Lösung wird in Infusionsflaschen von 1/2 Liter Inhalt abgefüllt.

Beispiel 4:

Wie in Beispiel 2 beschreiben wird aus

| | |
|---|---|
| alpha-Ketoglutarsäure | 12,000 g/l |
| 5-Hydroxymethylfurfural | 2,000 g/l |
| $CaCl_2.2H_2O$ | 0,883 g/l |
| KOH 85 %ig | 1,980 g/l |
| $MgCl_2.6H_2O$ | 1,220 g/l |
| NaOH | 2,800 g/l |
| $Na_2$-Glycerophosphat.$5H_2O$ | 9,184 g/l |
| $ZnCl_2$ | 0,019 g/l |
| Glucose | 20,000 g/l |

1 Liter einer Lösung bereitet, die die beiden Wirkstoffe im Verhältnis 6 : 1 enthält. Weiters sind Elektrolyte in folgender molarer Konzentration zugegen:

| | |
|---|---|
| Na | 130,0 mmol/l |
| K | 30,0 mmol/l |
| Ca | 6,0 mmol/l |
| Mg | 6,0 mmol/l |
| Zn | 0,1 mmol/l |
| Chlorid | 24,2 mmol/l |
| Phosphat | 20,0 mmol/l |

Der pH-Wert der Lösung beträgt 4,68. Sie wird in Infusionsflaschen von 1/2 Liter Inhalt abgefüllt.

Beispiel 5:

345,12 g alpha-Ketoglutarsäure-mono-Na-Salz, 0,8 g Zinkoxyd und 1204 g Staubzucker werden in einem Planetenmischer trocken gemischt und durch ein Sieb der Maschenweite 0,7 mm gesiebt. Das so erhaltene Material wird in den Mischer zurückgegeben und bei laufendem Mischwerk mit 100 g destilliertem Wasser versetzt und bis zur Agglomeratbildung gemischt. Nach Trocknung bei 50° C wird das Produkt durch ein Sieb der Maschenweite 1,25 mm granuliert und in einem Planetenmischer mit 50,0 g 5-Hydroxymethylfurfural gemischt. Der pH-Wert beträgt etwa 3. Man erhält 1600 g einer Masse, die als Trinkgranulat eingesetzt werden kann. Sie wird in Portionsbeutel zu je 4 g abgefüllt. Dieser Portionsbeutel enthält 0,75 g alpha-Ketoglutarsäure und 0,125 g 5-Hydroxymethylfurfural.

Beispiel 6:

1,2 g alpha-Ketoglutarsäure werden in Form des Mono-Na-Salzes mit 0,2 g 5-Hydroxymethylfurfural, 3,6 g Saccharose und 0,002 g Zinkoxyd gemischt und durch Zusatz von Wasser bis zu einem Volumen von 20 ml in Lösung gebracht. Diese Lösung dient zur Herstellung einer Trinkampulle.
Die Präparate gemäß den Beispielen 5 und 6 sind zur oralen Verabreichung, insbesondere zur Behandlung von Fällen mit Karcinom der oberen Verdauungsorgane wie des Magens vorgesehen.

Beispiel 7:

Die gleichen Stoffe wie in Beispiel 6, jedoch mit dem Unterschied, daß anstelle der Saccharose übliche Gleit- und Sprengmittel eingesetzt werden, werden zu Tabletten verarbeitet und anschließend mit einem magensaftresistenten Überzug versehen. Sie können zur Behandlung von Tumoren des Dünndarms dienen.

Beispiel 8:

15 g Methylzellulose, die in Form eines flüssigen Schleimes vorliegt, werden mit 6 g alpha-Ketoglutarsäure, 1,2 g NaOH, 0,726 g 85 %iger KOH, 16 g $NaH_2PO_4.2H_2O$ und 6 g $Na_2HPO_4.12H_2O$ und schließlich mit 1 g 5-Hydroxymethylfurfural gemischt.
Die resultierende Lösung einer Viskosität von 20-50 cp und einem pH-Wert von etwa 6 wird auf 1 l aufgefüllt. Sie kann in Portionen von 1/4 Liter als Klistier verabreicht werden und dient bevorzugt zur Behandlung von Kolonkarczinomen, wobei die Tagesdosis 2 Klistiere beträgt.

Beispiel 9:

Ein 33jähriger männlicher Patient mit einem hühnereigroßen, inoperablen Bronchialkarzinom mit infauster Prognose erhielt über 30 Tage täglich 1/2 Liter der Lösung gemäß Beispiel 2 infundiert, entsprechend einer Tagedosis an alpha-Ketoglutarsäure von 3 g und einer solchen an 5-Hydroxymethylfurfural von 1 g. Nach Ablauf der 30 Tage war der Tumor röntgenologisch nicht mehr sichtbar. Der vorher stimmungsmäßig depressiv eingestellte Patient wurde wieder positiv gestimmt und entwickelte einen lebhaften Appetit.

Beispiel 10:

Ein männlicher Patient mit 78 Jahren, der an einem rezidivierenden Blasenkarzinom mit Lungenmetastasen erkrankt war, erhielt über 30 Tage je 1/2 Liter der Lösung gemäß Beispiel 2 infundiert. Danach waren weder der Blasentumor noch die Lungenmetastasen röntgenologisch nachweisbar.

Beispiel 11:

Einer 70 Jahre alten Patientin wurde ein ulzerierendes Mammakarzinom mit mutiplen lokalen Metastasen chirurgisch entfernt. Da sich unmittelbar nach der Operation wieder Metastasen ausbildeten, wurde ihr täglich 1/2 Liter der Lösung gemäß Beispiel 2 infundiert. Diese Infusionsbehandlung mußte nach 7 Tagen wegen Venenunverträglichkeit abgebrochen werden. Da sich kurze Zeit nach Abheilung des Operationsfeldes im verheilten Operationsgebiet eine Metastase in Form eines harten Knotens bildete, wurde die Infusionsbehandlung unter Beachtung größter Sorgfalt bei der Verabreichung wieder aufgenommen.
Nach 7 Infusionen von je 1/2 Liter der Lösung gemäß Beispiel 2 war der harte Knoten verflüssigt, nach weiteren 7 Infusionen war das Gebiet des ursprünglichen harten Knotens unauffällig. Die Infusionsbehandlung wurde sicherheitshalber 6 Tage fortgesetzt.

Beispiel 12:

Ein 87 Jahre alter Patient, der an einem primären Prostatakarzinom mit Lungenmetastasen litt, die häufig Bluthusten und Atembeschwerden verursachten, erhielt täglich über 16 Tage 1/2 Liter der Lösung gemäß Beispiel 2. Bei der dann vorgenommenen Zwischenuntersuchung hatte die Prostata wieder die normale Konsistenz und die Rundherde in der Lunge waren am Röntgenschirm nicht mehr sichtbar. Der Patient hatte keinen Hustenreiz mehr und konnte nach seiner Angabe wieder leicht atmen.

Beispiel 13:

Bei einem Patienten mit Morbus Hodgkin (Lymphogranulomatose) im Stadium IV mit infauster Prognose wurde nach 15 Infusionen von je 1/2 Liter der Lösung nach Beispiel 2 folgender Zwischenbefund erhoben: von den angeschwollenen Lymphknoten an Hals, Achselhöhlen und Leisten waren die Achsellymphknoten abgeschwollen, die übrigen Lymphknoten waren noch geschwollen, der Arzt stellte jedoch eine Besserung des Gesamtzustandes des Patienten fest.

Beispiel 14:

Ein Patient, bei dem bei einer Nachuntersuchung nach einem Magengeschwür über eine Gastroskopie eine ausgeprägte Präcancerose festgestellt wurde und dem eine weitgreifende Magenteilresektion empfohlen wurde, erhielt über 4 Tage 3 x täglich eine Portion des Trinkgranulates gemäß Beispiel 5, das in Wasser aufgeschlämmt wurde, in Form einer Rollkur verabreicht. Bei der am 4. Tag vorgenommenen Nachuntersuchung, bei der nocheinmal Proben der Magenschleimhaut entnommen wurden, konnte keine Malignität im zytologisch untersuchten Gewebe mehr festgestellt werden. Der Patient setzte daraufhin die Behandlung mit der gleichen Tagesdosis an dem Trinkgranulat gemäß Beispiel 5 noch 6 Wochen hindurch fort. Bei der anschließenden zweiten Nachuntersuchung konnte bei den entnommenen und zytologisch geprüften Proben der Magenschleimhaut ebenfalls keine Malignität mehr festgestellt werden.

Beispiel 15:

Eine 79jährige Patientin mit einem Mammakarzinom, das palliativ operiert wurde, zeigte nach erfolgter Operation eine Lymphknotenschwellung, die die Absiedlung von Fernmetastasen anzeigte. Bei schlechtem Allgemeinzustand und Gewichtsverlust wurde mit einer Infusionstherapie unter Verwendung der Lösung gemäß Beispiel 2 bei einer Tagesdosis von 1 g 5-Hydroxymethylfurfural und 3 g alpha-Ketoglutarsäure begonnen. Nach der 6. Infusion war eine deutliche Besserung zu verzeichnen. Nach der Verabfolgung von insgesamt 14 Infusionen wurde dann auf eine orale Weiterbehandlung umgestellt, bei der die Patientin täglich 3 Portionen des Trinkgranulates gemäß Beispiel 5, das sind also 2,25 g alpha-Ketoglutarsäure und 0,375 g 5-Hydroxymethylfurfural pro Tag, erhielt.
Diese orale Therapie wurde 6 Monate fortgesetzt. Bei einer darauffolgenden Kontrolluntersuchung war die Lymphknotenschwellung nicht mehr tastbar und auch im Röntgenbild nicht mehr nachweisbar. Die Patientin hatte eine Gewichtszunahme zu verzeichnen und zeigte ein wesentlich gebessertes Allgemeinbefinden.
Prinzipiell können auch alle anderen bösartigen Tumorerkrankungen, die mit einer Erhöhung der alpha-Ketoglutarsäure im Serum verbunden sind, auf gleiche Weise behandelt werden.

**Patentansprüche**

**Patentansprüche für folgende Vertragsstaaten : AT, BE, CH, LI, DE, FR, GB, IT, LU, NL, SE**

1. Mittel mit zerstörender Wirkung auf maligne Tumore, dadurch gekennzeichnet, daß es als Wirkstoffe alpha-Ketoglutarsäure und eine oder mehrere zur Azomethinbildung befähigte Verbindungen aus der Gruppe 5-Hydroxymethylfurfural, Dehydroascorbinsäure, Maltol und Vanillin enthält.
2. Mittel nach Anspruch 1, dadurch gekennzeichnet, daß die alphaKetoglutarsäure zur zur Azomethinbildung befähigten Verbindung in einem Gewichtsverhältnis von 2 : 1 bis 12 : 1 vorliegt.
3. Mittel nach Anspruch 2, dadurch gekennzeichnet, daß das Gewichtsverhältnis 3 : 1 bis 8 : 1 beträgt.
4. Mittel nach einem der Ansprüche 1 bis 3, dadurch gekennzeichnet, daß es außerdem Glucose oder Fructose enthält.
5. Mittel nach einem der Ansprüche 1 bis 4, dadurch gekennzeichnet, daß es außerdem Elektrolyte aus der Gruppe Natrium-, Kalium-, Calcium-, Magnesium- und Zinkion, zusammen mit Chloridion und Phosphation als Anionen enthält.
6. Mittel nach einem der Ansprüche 1 bis 5, dadurch gekennzeichnet,daß es in einer festen oder flüssigen, oral zu verabreichenden Darreichungsform vorliegt, die die alpha-Ketoglutarsäure zumindest zum Teil in Form ihres Mono-Na- oder Mono-K-Salzes enthält.
7. Verfahren zur Herstellung eines Mittels gemäß einem der Ansprüche 1-6, dadurch gekennzeichnet, daß die alpha-Ketoglutarsäure und die zur Azomethinbildung befähigte Verbindung unter Mischung in eine zur intravenösen, oralen oder rectalen Verabreichung oder für die äußerliche Anwendung geeignete Form gebracht, und vor, während oder nach der Mischung der Wirkstoffe der pH-Wert durch Zusatz von Alkalien, nicht aber von Ammoniak oder Aminen, auf den physiologisch verträglichen Bereich eingestellt wird.
8. Verfahren nach Anspruch 7 zur Herstellung einer insbesondere zur intravenösen Verabreichung geeigneten Lösung, dadurch gekennzeichnet, daß die alpha-Ketoglutarsäure sowie gegebenenfalls gewünschte weitere Zusätze wie Glucose, Fructose oder Elektrolyte in Wasser gelöst werden, in der resultierenden Lösung der pH-Wert mittels Alkalien auf 4-6 eingestellt und anschließend die zur Azomethinbildung befähigte Verbindung zugemischt wird.
9. Verfahren nach Anspruch 7 zur Herstellung einer zur oralen oder rectalen Verabreichung geeigneten Präparation, dadurch gekennzeichnet, daß die alpha-Ketoglutarsäure durch Zusatz von Alkalien zumindest zum Teil in eines ihrer Monosalze übergeführt und anschließend die zur Azomethinbildung befähigte Verbindung zugefügt wird.
10. Präparation zur Anwendung in der Therapie von Krebskranken, dadurch gekennzeichnet, daß sie als Wirkstoffe alpha-Ketoglutarsäure und eine oder mehrere zur Azomethinbildung befähigte Verbindungen aus

EP 0 326 826 B1

der Gruppe 5-Hydroxymethylfurfural, Dehydroascorbinsäure, Maltol und Vanillin enthält.

**Patentansprüche für folgende Vertragsstaaten: ES, GR**

1. Verfahren zur Herstellung eines Mittels mit zerstörender Wirkung auf maligne Tumore, dadurch gekennzeichnet, daß alpha-Ketoglutarsäure und eine oder mehrere zur Azomethinbildung befähigte Verbindungen aus der Gruppe 5-Hydroxymethylfurfural, Dehydroascorbinsäure, Maltol und Vanillin unter Mischung in eine für die intravenöse, orale oder rectale Verabreichung oder für die äußerliche Anwendung geeignete Form gebracht werden, und vor, während oder nach der Mischung der Wirkstoffe der pH-Wert durch Zusatz von Alkalien, nicht aber von Ammoniak oder Aminen, auf den physiologisch verträglichen Bereich eingestellt wird.

2. Verfahren nach Anspruch 1, dadurch gekennzeichnet, daß die alpha-Ketoglutarsäure mit der zur Azomethinbildung befähigten Verbindung in einem Gewichtsverhältnis von 2 : 1 bis 12 : 1 gemischt wird.

3. Verfahren nach Anspruch 2, dadurch gekennzeichnet, daß die Mischung in einem Gewichtsverhältnis von 3 : 1 bis 8 : 1 vorgenommen wird.

4. Verfahren nach einem der Ansprüche 1 bis 3, dadurch gekennzeichnet, daß außerdem Glucose oder Fructose zugesetzt werden.

5. Verfahren nach einem der Ansprüche 1 bis 4, dadurch gekennzeichnet, daß außerdem Elektrolyte aus der Gruppe Natrium-, Kalium-, Calcium-, Magnesium- und Zinkion zusammen mit Chloridion und Phosphation als Anionen zugesetzt werden.

6. Verfahren nach einem der Ansprüche 1 bis 5, dadurch gekennzeichnet, daß zur Herstellung einer festen oder flüssigen, oral zu verabreichenden Darreichungsform die alpha-Ketoglutarsäure zumindest zum Teil in Form ihres Mono-Na- oder Mono-K-Salzes eingesetzt wird.

7. Verfahren nach einem der Ansprüche 1 bis 6, dadurch gekennzeichnet, daß insbesondere zur Herstellung einer zur intravenösen Verabreichung geeigneten Lösung die alpha-Ketoglutarsäure sowie gegebenenfalls gewünschte weitere Zusätze wie Glucose, Fructose oder Elektrolyte in Wasser gelöst werden, in der resultierenden Lösung der pH-Wert mittels Alkalien auf 4-6 eingestellt und anschließend die zur Azomethinbildung befähigte Verbindung zugemischt wird.

**Claims**

**Claims for the following Contracting States : AT, BE, CH, LI, DE, FR, GB, IT, LU, NL, SE**

1. An agent having a destructive action on malignant tumors, which contains as active substances alpha-ketoglutaric acid and one or more compounds capable of azomethine formation, selected from the group comprising 5-hydroxymethylfurfural, dehydroascorbic acid, maltol and vanillin.

2. An agent as claimed in claim 1, wherein the ratio by weight of alpha-ketoglutaric acid to the compound capable of azomethine formation is 2:1 to 12:1.

3. An agent as claimed in claim 2, wherein the ratio by weight is 3:1 to 8:1.

4. An agent as claimed in any of claims 1 to 3, which additionally contains glucose or fructose.

5. An agent as claimed in any of claims 1 to 4, which additionally contains electrolytes selected from the group comprising the sodium, potassium, calcium, magnesium and zinc ion, together with the chloride ion and phosphate ion as anions.

6. An agent as claimed in any of claims 1 to 5, which is present in a solid or liquid administration form to be administered orally, which contains the alpha-ketoglutaric acid at least in part in the form of its mono-Na or mono-K salt.

7. A method for the production of an agent as claimed in any of claims 1-6, which comprises bringing the alpha-ketoglutaric acid and the compound capable of azomethine formation into a form suitable for intravenous, oral or rectal administration or for topical application by mixing, and adjusting the pH to the physiologically tolerable range by addition of alkalis, but not of ammonia or amines, before, during or after the mixing of the active substances.

8. The method as claimed in claim 7 for the production of a solution which is in particular suitable for intravenous administration, wherein the alpha-ketoglutaric acid and, if appropriate, desired further additives such as glucose, fructose or electrolytes are dissolved in water, the pH in the resulting solution is adjusted to 4-6 by means of alkalis and the compound capable of azomethine formation is then admixed.

9. The method as claimed in claim 7 for the production of a preparation suitable for oral or rectal administration, wherein the alpha-ketoglutaric acid is converted at least in part into one of its mono salts by addition of alkalis and the compound capable of azomethine formation is then added.

10

10. A preparation for use in the therapy of cancer patients, which as active substances contains alphaketoglutaric acid and one or more compounds capable of azomethine formation, selected from the group comprising 5-hydroxymethylfurfural, dehydroascorbic acid, maltol and vanillin.

**Claims for the following Contracting States : ES, GR**

1. A method for the production of an agent having a destructive action on malignant tumors, which comprises bringing alpha-ketoglutaric acid and one or more compounds capable of azomethine formation, selected from the group comprising 5-hydroxymethylfurfural, dehydroascorbic acid, maltol and vanillin into a form suitable for intravenous, oral or rectal administration or for topical application by mixing, and adjusting the pH to the physiologically tolerable range by addition of alkalis, but not of ammonia or amines, before, during or after the mixing of the active substances.

2. The method as claimed in claim 1, wherein the alpha-ketoglutaric acid is mixed with the compound capable of azomethine formation in a ratio by weight of 2:1 to 12:1.

3. The method as claimed in claim 2, wherein the mixing is carried out in a ratio by weight of 3:1 to 8:1.

4. The method as claimed in any of claims 1 to 3, wherein glucose or fructose is additionally added.

5. The method as claimed in any of claims 1 to 4, wherein electrolytes selected from the group comprising the sodium, potassium, calcium, magnesium and zinc ion, together with the chloride ion and phosphate ion are additionally added as anions.

6. The method as claimed in any of claims 1 to 5, wherein the alpha-ketoglutaric acid is employed at least in part in the form of its mono-Na or mono-K salt for the production of a solid or liquid administration form to be administered orally.

7. The method as claimed in any of claims 1 to 6, wherein, in particular for the production of a solution suitable for intravenous administration, the alpha-ketoglutaric acid and, if appropriate, desired further additives such as glucose, fructose or electrolytes are dissolved in water, the pH in the resulting solution is adjusted to 4-6 by means of alkalis and the compound capable of azomethine formation is then admixed.


**Revendications**


**Revendications pour les Etats contractants suivants : AT, BE, CH, LI, DE, FR, GB, IT, LU, NL, SE**

1. Agent à effet destructeur sur des tumeurs malignes, caractérisé par le fait qu'il contient, comme principes actifs, de l'acide alpha-cétoglutarique et un ou plusieurs composés susceptibles de former de l'azométhine, choisis dans le groupe constitué de 5-hydroxyméthylfurfural, acide deshydro-ascorbique, maltol et vanilline.

2. Agent suivant la revendication 1, caractérisé par le fait que l'acide alpha-cétoglutarique se trouve par rapport au composé susceptible de former de l'azométhine en un rapport en poids de 2/1 à 12/1.

3. Agent suivant la revendication 2, caractérisé par le fait que le rapport en poids est compris entre 3/1 et 8/1.

4. Agent suivant l'une des revendications 1 à 3, caractérisé par le fait qu'il contient en outre du glucose ou du fructose.

5. Agent suivant l'une des revendications 1 à 4, caractérisé par le fait qu'il contient en outre des électrolytes choisis dans le groupe constitué des ions sodium, potassium, calcium, magnésium et zinc, simultanément avec les ions chlorure et phosphate en tant qu'anions.

6. Agent suivant l'une des revendications 1 à 5, caractérisé par le fait qu'il se trouve sous une forme solide ou liquide à administrer par voie buccale, qui contient l'acide alpha-cétoglutarique au moins en partie sous forme de son sel mono-sodique ou mono-potassique.

7. Procédé de préparation d'un agent selon l'une des revendications 1 à 6, caractérisé par le fait que l'acide alpha-cétoglutarique et le composé susceptible de former de l'azométhine est amené, avec mélange, sous une forme appropriée à l'administration intraveineuse, buccale ou rectale ou à l'application externe, et qu'avant, pendant ou après le mélange des principes actifs, le pH, par addition d'alcalis, mais non pas d'ammoniaque ou d'amines, est réglé à une valeur physiologiquement acceptable.

8. Procédé suivant la revendication 7 pour la préparation d'une solution appropriée en particulier à l'administration intraveineuse, caractérisé par le fait que l'acide alpha-cétoglutarique, ainsi que d'autres additifs éventuellement souhaités, tels que glucose, fructose ou électrolytes, sont dissous dans de l'eau, le pH dans la solution résultante est réglé à une valeur de 4 à 6 au moyen d'alcalis, puis le composé susceptible de former de l'azométhine est ajouté avec mélange.

9. Procédé suivant la revendication 7 pour l'obtention d'une préparation appropriée à l'administration buccale ou rectale, caractérisé par le fait que l'acide alpha-cétoglutarique, par addition d'alcalis, est transformé au moins en partie en un de ses mono-sels, après quoi on ajoute le composé susceptible de former de l'azométhine.

10. Préparation pour l'utilisation dans la thérapie des cancéreux, caractérisée par le fait qu'elle contient, comme principes actifs, de l'acide alpha-cétoglutarique et un ou plusieurs composés susceptibles de former de l'azométhine, choisis dans le groupe constitué de 5-hydroxyméthylfurfural, acide déshydro-ascorbique, maltol et vanilline.

**Revendications pour les Etats contractants suivants : ES, GR**

1. Procédé de préparation d'un agent à effet destructeur sur des tumeurs malignes, caractérisé par le fait que de l'acide alpha-cétoglutarique et un ou plusieurs composés susceptibles de former de l'azométhine, choisis dans le groupe constitué de 5-hydroxyméthylfurfural, acide deshydro-ascorbique, maltol et vanilline sont amenés, avec mélange, sous une forme appropriée à l'administration intraveineuse, buccale ou rectale ou à l'application externe, et qu'avant, pendant ou après le mélange des principes actifs, le pH, par addition d'alcalis, mais ,non pas d'ammoniaque ou d'amines, est réglé à une valeur physiologiquement acceptable.

2. Procédé selon la revendication 1, caractérisé par le fait que l'acide alpha-cétoglutarique se trouve par rapport au composé susceptible de former de l'azométhine en un rapport en poids de 2/1 à 12/1.

3. Procédé selon la revendication 2, caractérisé par le fait que le mélange est effectué dans un rapport en poids compris entre 3/1 et 8/1.

4. Procédé selon l'une des revendications 1 à 3, caractérisé par le fait qu'on ajoute en outre du glucose ou du fructose.

5. Procédé selon l'une des revendicaitons 1 à 4, caractérisé par le fait qu'on ajoute en outre des électrolytes choisis dans le groupe constitué des ions sodium, potassium, calcium, magnésium et zinc, simultanément avec les ions chlorure et phosphate en tant qu'anions.

6. Procédé selon l'une des revendications 1 à 5, caractérisé par le fait que, pour obtenir une forme solide ou liquide à administrer par voie buccale, on ajoute l'acide alpha-cétoglutarique au moins en partie sous forme de son sel mono-sodique ou mono-potassique.

7. Procédé selon l'une des revendications 1 à 6, caractérisé par le fait qu'en particulier pour la préparation d'une solution appropriée en particulier à l'administration intraveineuse, l'acide alpha-cétoglutarique, ainsi que d'autres additifs éventuellement souhaités, tels que glucose, fructose ou électrolytes, sont dissous dans de l'eau, le pH dans la solution résultante est réglé à une valeur de 4 à 6 au moyen d'alcalis, puis le composé susceptible de former de l'azométhine est ajouté avec mélange.